# EUROPÄISCHE PATENTANMELDUNG

(11) **EP 1 277 460 A2**
(43) Veröffentlichungstag der Anmeldung: **22.01.2003**
(21) Anmeldenummer: 02015838.2
(22) Anmeldetag: 16.07.2002
(51) Int. Cl.: A61K 7/42

(54) **Kosmetische Formulierungen mit Benzoazolyl-, Benzodiazolyl-, bzw. Benzotriazol-Lichtschutzfiltern und Dihydroxyaceton (DHA)**

(30) Priorität: 18.07.2001 DE 10135024
(71) Anmelder: Beiersdorf AG, 20245 Hamburg (DE)
(72) Erfinder: Knüppel, Anja, 22527 Hamburg (DE); Eitrich, Anja, 22587 Hamburg (DE)

(57) **Zusammenfassung**

Die vorliegende Erfindung betrifft kosmetische Formulierungen, die mindestens einen UV-Filter mit Benzoazolyl- oder Benzodiazolyl-Gruppen und/oder mindestens einen UV-Filter auf Basis eines Benzotriazolderivates und mindestens eine Selbstbräunungssubstanz (1,3-Dihydroxyaceton u.a.) enthalten.

## Beschreibung

Die vorliegende Erfindung betrifft kosmetische Formulierungen, die mindestens einen UV-Filter mit Benzoazolyl- und/oder mindestens einen UV-Filter mit Benzodiazolyl-Gruppen und/oder mindestens einen UV-Filter auf Basis eines Benzotriazolderivates und mindestens eine Selbstbräunungssubstanz (z.B.: 1,3-Dihydroxyaceton) enthalten.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.
Zum Schutz der Haut wurden daher eine Reihe von Lichtschutzfiltersubstanzen entwickelt, die in kosmetischen Zubereitungen eingesetzt werden können. Diese UVAund UVB-Filter sind in den meisten Industrieländern in Form von Positivlisten wie dem Anlage 7 der Kosmetikverordnung zusammengefasst.

Zu den eingesetzten UV-Lichtschutzfiltern zählt unter anderem 2-Hydroxy-4-methoxybenzophenon. Dieser UV-Breitbandfilter ist unter den Handelsnamen Eusolex 4360 (Merck), Neo Heliopan BB (H&R), Escalol 567 (ISP) und Uvasorb MET/C (Sigma) kommerziell erhältlich.

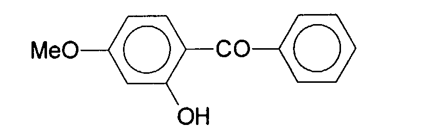

Desweiteren wird 1-(4-*tert*.-Butylphenyl)-3-(4'-methoxyphenyl)-propan-1,3-dion als UVA-Schutzfilter eingesetzt [Eusolex 9020 (Merck), Parsol 1789 (Givaudan)].

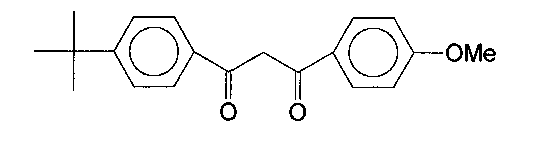

Weitere in der Praxis verwendete Sonnenschutzfilter sind unter anderem 4-Methoxyzimtsäure-2-ethylhexylester [Escalol 557 (ISP), Eusolex 2292 (Merck), Neo Heliopan AV (H&R), Parsol MCX (Givaudan)] 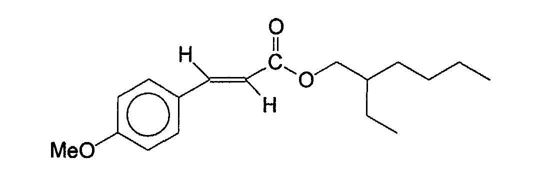 sowie 3-(4'-Methyl)benzyliden-bornan-2-on [Eusolex 6300(Merck), Neo Heliopan MBC (H&R), Parsol 5000 (Givaudan)] 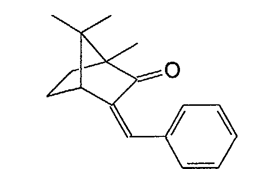 und das symmetrisch substituierte 4,4',4"-(1,3,5-Triazin-2,4,6-triyltriimino)-tris-benzoesäure-tris(2-ethylhexylester), synonym: 2,4,6-Tris-[anilino-(p-carbo-2'-ethyl-1'-hexyloxy)]-1,3,5-triazin. [UVINUL T 150 (BASF)].

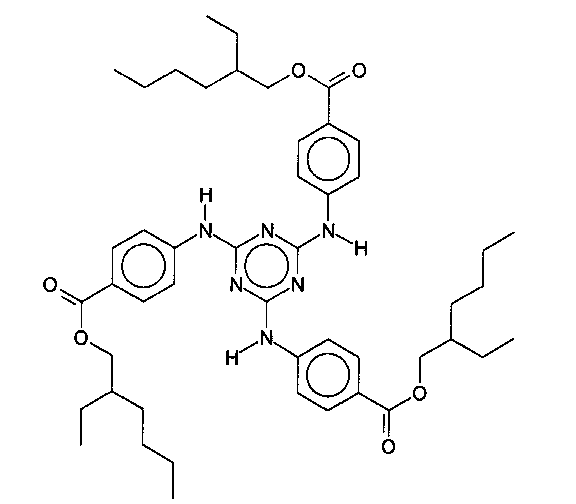

Eine außerordentlich bedeutsame Klasse an UV-Lichtschutzfiltern leitet sich von den Benzimidazolsulfonsäuren ab.

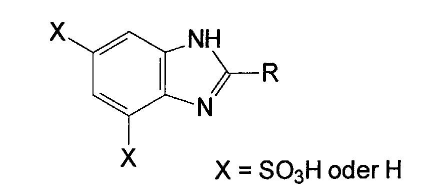

Eine weitere Klasse vorteilhafter Lichtschutzfilter baut auf dem Strukturelement des Benzotriazols auf.

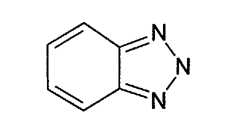

Den natürlichen Schutz vor den negativen Folgen der Sonnenstrahlung bietet die Bräunung (Pigmentierung) der Haut. Die Epidermis enthält in ihrer untersten Schicht, der Basalschicht, neben den Basalzellen einzelne pigmentbildende Zellen, die Melanocyten. Durch UV-Licht wird in diesen Zellen die Produktion von Melanin angeregt, das in die Kerantionocycten (Hornzellen) transportiert und dort als braune Hautfarbe sichtbar wird.

Diese von der Aminosäure Tyrosin ausgehende Pigmentbildung wird überwiegend durch UVB-Strahlung initiiert und als "indirekte Pigmentierung" bezeichnet. Ihre Entwicklung läuft über mehrere Tage; die so erhaltene Sonnenbräune besteht über einige Wochen. Bei der "Direkt-Pigmentierung", die mit der Sonnenbestrahlung einsetzt, werden vorwiegend farblose Melanin-Vorstufen durch UVA-Strahlung zu dunkel gefärbten Melanin oxidiert. Da diese Oxidierung reversibel ist, führt sie zu einer nur kurz anhaltenden Hautbräunung.

Eine künstliche Bräunung der Haut lässt sich äußerlich mit Hilfe von Schminke und oral durch Einnahme von Carotinoiden erzeugen.

Weitaus beliebter jedoch ist die künstliche Bräunung der Haut, welche sich durch Auftragen von sogenannten Selbstbräunern erzielen lässt. Diese Verbindungen weisen als chemisches Strukturmerkmal Keto- bzw. Aldehydgruppen in Nachbarschaft zu Alkoholfunktionen auf. Diese Ketole bzw. Aldole gehören überwiegend zur Substanzklasse der Zucker. Der am häufigsten verwendete Selbstbräuner ist das 1,3-Dihydroxyaceton.

Die Verbindungen können mit den Proteinen und Aminosäuren der Hornschicht der Haut im Sinne einer Maillard-Reaktion umgesetzt werden, wobei über einen noch nicht vollständig aufgeklärten Reaktionsweg Polymerisate entstehen, die der Haut einen bräunlichen Farbton verleihen. Diese Reaktion ist nach etwa 4 bis 6 Stunden abgeschlossen; die so erzielte Bräune ist nicht abwaschbar und wird erst mit der normalen Hautabschuppung entfernt.

Im Gegensatz zur natürlichen Pigment-Bräunung (Sonnenbräunung) bietet die mittels Selbstbräuner erzielte Bräunung jedoch keinerlei Schutz vor UV-Strahlung. Ein weiterer Nachteil von 1,3-Dihydroxyaceton besteht darin, dass es, insbesondere unter dem Einfluss ultravioletter Strahlung, wenn auch in meist geringen Mengen Formaldehyd abspaltet. Auch anorganische Eisenoxide (z.B. Fe₂O₃, FeO) bewirken eine extrem schnelle Zersetzung des DHA und Verfärbung der kosmetischen Formulierung. Ferner sind zur Stabilisierung von DHA saure Medien (pH-Wert < 6) vonnöten. Es gelang bisher lediglich die Kombination von 1,3-Dihydroxyaceton mit Hilfe von unpolaren, öllöslichen UV-Filtern wie Dibenzoylmethanderivaten, Methylencampherderivaten oder Triazinderivaten.
Um diesen Nachteilen des Standes der Technik abzuhelfen, war also Aufgabe der vorliegenden Erfindung.

Es war überraschend und für den Fachmann nicht voraus zusehen, dass kosmetische Formulierungen, die mindestens einen UV-Filter mit Benzoazolyl- und/oder mindestens einen UV-Filter mit Benzodiazolyl-Gruppen und/oder mindestens einen UV-Filter auf Basis eines Benzotriazolderivates und mindestens eine Selbstbräunungssubstanz enthalten, den Nachteilen des Standes der Technik abhelfen.
Als Selbstbräuner werden erfindungsgemäß vorteilhaft unter anderem eingesetzt:

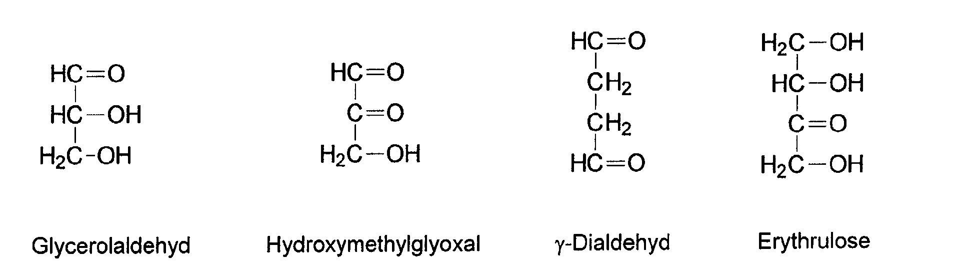

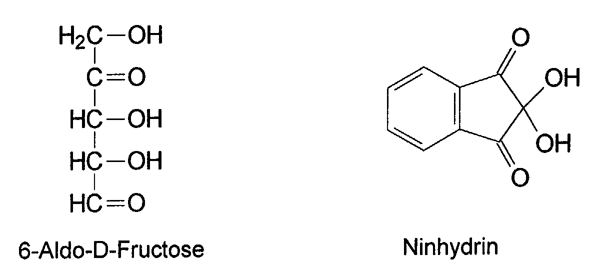

Ferner ist das 5-Hydroxy-1,4-naphtochinon (Juglon) zu nennen, das aus den Schalen frischer Walnüsse extrahiert wird 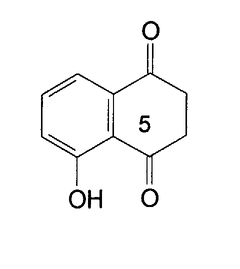 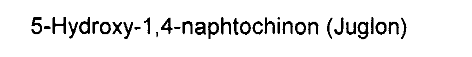 sowie das in den Henna-Blättern vorkommende 2-Hydroxy-1,4-naphtochinon (Lawson).

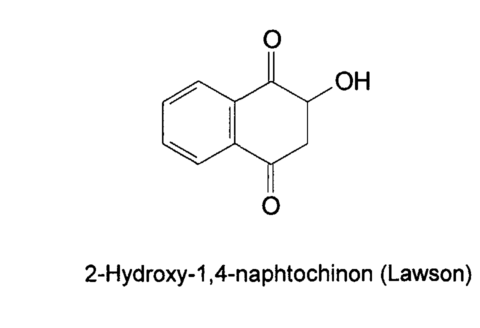

Ganz besonders bevorzugt im Sinne der Erfindung ist das 1,3-Dihydroxyaceton (DHA), ein im menschlichen Körper vorkommender dreiwertiger Zucker.

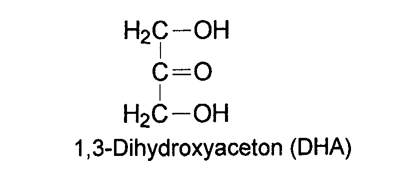

Durch die vorliegende Erfindung wurde die Stabilität von 1,3-Dihydroxyaceton enthaltenen kosmetischen Formulierungen im Vergleich zum Stand der Technik entscheidend erhöht.

UV-Filter mit Benzoazolyl- oder Benzodiazolyl-Gruppen lassen sich überraschenderweise schon ab einem pH-Wert von pH 5 in ihre wasserlöslichen Salze überführen. Damit lassen sie sich in nicht vorher gesehener Weise in Selbstbräunungsformulierungen, die 1,3-Dihydroxyaceton enthalten können, einarbeiten.
UV-Filter aus der Gruppe der Benzotriazole können in gelöster oder in unlöslicher pigmentärer Form in 1,3-Dihydroxyaceton enthaltene kosmetische Formulierungen eingearbeitet werden, ohne dass es zu einer Verfärbung der Emulsion und einer schnellen Zersetzung des Selbstbräuners kommt. Durch den Einsatz dieser vor allem pigmentären Filter ist es darüber hinaus möglich, sehr hohe Lichtschutzfaktoren zu erreichen.
Erfindungsgemäße kosmetische Zubereitungen enthalten also vorteilhafter Weise mindestens einen UV-Filter mit Benzoazolyl- und/oder Benzodiazolyl-Gruppen und/oder mindestens einen UV-Filter auf Basis eines Benzotriazolderivates.
Hierzu zählen die im Sinne der Erfindung besonders vorteilhafte 2-Phenylbenzimidazol-5-sulfonsäure und ihre Natrium-, Kalium- und Triethanolammoniumsalze [Eusolex 232 (Merck), Neo Heliopan Hydro (H&R), Parsol HS (Givaudan)] 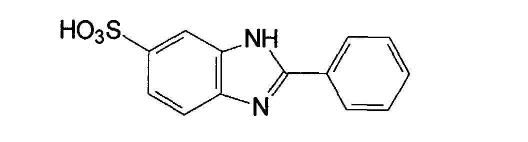 sowie das Bisimidazolidat Phenylen-1,4-bis-(2-benzimidazyl)-3,3',5,5'-tetrasulfonsäure und ihre Natrium-, Kalium- und Triethanolammoiumsalze, insbesondere das DiNatriumsalz [Neo Heliopan AP (Haarmann & Reimer)].

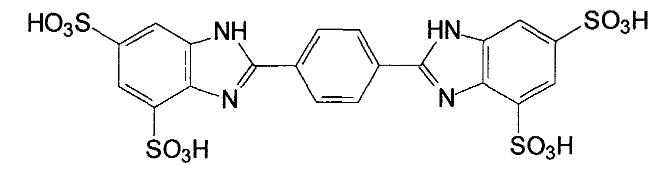

Besonders vorteilhafte Lichtschutzfilter im Sinne der vorliegenden Erfindung, die sich durch das Strukturmotiv des Benzotriazols auszeichnen, werden durch die Struktur 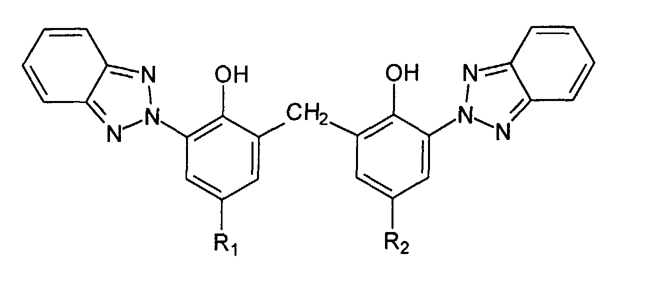 wiedergegeben. R₁ und R₂ können unabhängig voneinander aus der Gruppe der verzweigten oder unverzweigten C₁-C₁₈-Alkylreste gewählt werden, die gegebenenfalls mit einer oder mehreren C₁-C₄- Alkylgruppen, C₅-C₁₂-Cycloalkyl- oder Arylresten substituiert sind.

Das bevorzugte Benzotriazolderivat ist das 2,2'-Methylen-bis-(6-(2H-benzotriazol-2-yl)-4-(1,1,3,3-tetramethylbutyl)-phenol) [Tinosorb M (Ciba)], welches durch die chemische Strukturformel 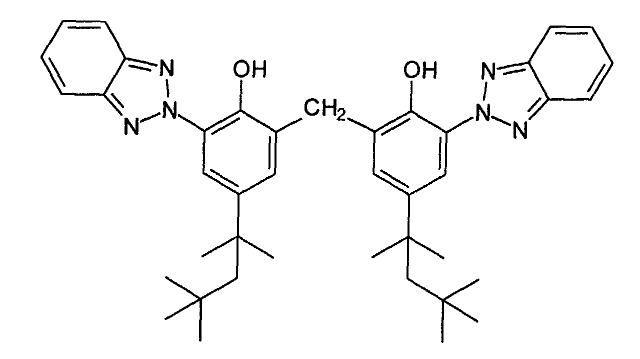 gekennzeichnet ist.

Vorteilhafter Breitbandfilter im Sinne der vorliegenden Erfindung ist ferner das 2-(2Hbenzotriazol-2-yl)-4-methyl-6-[2-methyl-3- [1,3,3,3 -tetramethyl-1 -[(trimethylsilyl)oxy]disiloxanyl]propyl]-phenol (CAS-Nr.: 155633-54-8) [Mexoryl XL (Chimex)] mit der INCI-Bezeichnung Drometrizole Trisiloxane, welches durch die chemische Strukturformel 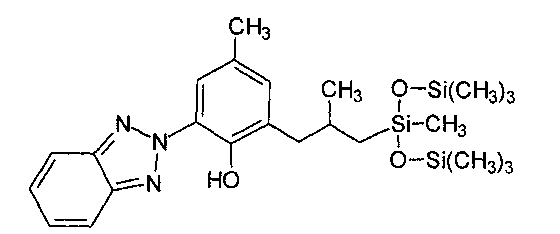 gekennzeichnet ist.

Die erfindungsgemäßen Lichtschutzfilter werden bevorzugt in einer Konzentration von 0,1 bis 10 Gewichts-%, insbesondere in einer Konzentration von 0,5 bis 5 Gewichts-%, bezogen auf das Gesamtgewicht der Formulierung, eingesetzt. Vorteilhaft im Sinne der Erfindung ist dabei der Einsatz des/der Benzotriazolderivate in pigmentärer Form. Darüber hinaus können noch weitere organische und/oder anorganische UV-Filter für die kosmetische Formulierung verwendet werden. Des Weiteren enthalten sie vorteilhafter Weise mindestens eine Selbstbräunungssubstanz, wobei 1,3-Dihydroxyaceton besonders vorteilhaft ist. Von Vorteil ist dabei eine Konzentration von 0,5 bis 10 Gewichts-% 1,3-Dihydroxyaceton und besonders eine Konzentration von 1 bis 7 Gewichts-%, bezogen auf das Gesamtgewicht der Formulierung.

Erfindungsgemäß können die kosmetischen und/oder dermatologischen Lichtschutzformulierungen wie üblich zusammengesetzt sein und dem kosmetischen und/oder dermatologischen Lichtschutz, ferner zur Behandlung, der Pflege und der Reinigung der Haut und/oder der Haare und als Schminkprodukt in der dekorativen Kosmetik dienen.

Zur Anwendung werden die kosmetischen und dermatologischen Zubereitungen erfindungsgemäß in der für Kosmetika üblichen Weise auf die Haut und/oder die Haare in ausreichender Menge aufgebracht.

Besonders bevorzugt sind solche kosmetischen und dermatologischen Zubereitungen, die in der Form eines Sonnenschutzmittels vorliegen. Vorteilhaft können diese zusätzlich mindestens einen weiteren UVA-Filter und/oder mindestens einen weiteren UVB-Filter enthalten.

Die kosmetischen und dermatologischen Zubereitungen können erfindungsgemäß kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente (z.B. TiO₂, ZnO, BaSO₄), die eine färbende Wirkung haben, Verdickungsmittel, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α -Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoëharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,001 bis 10 Gew.-%, besonders bevorzugt 0,05 - 7 Gew.-%, insbesondere 0,5 - 5 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 5 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 5 Gew.-%, bezogen auf das Gesamtgewicht der Formulierung, zu wählen.

Darüber hinaus eignen sich ausgewählte erfindungsgemäße Rezepturen, welche z. B. bekannte Antifaltenwirkstoffe wie Flavonglycoside (insbesondere α-Glycosylrutin), Coenzym Q10, Vitamin E und/oder Derivate und dergleichen enthalten, insbesondere vorteilhaft zur Prophylaxe und Behandlung kosmetischer oder dermatologischer Hautveränderungen, wie sie z. B. bei der Hautalterung (z.B. Falten und Fältchen) auftreten. Weiterhin vorteilhaft eignen sie sich gegen das Erscheinungsbild der trockenen bzw. rauhen Haut.

Eine gegebenenfalls vorhandene Lipidphase kann vorteilhaft gewählt werden aus folgender Substanzgruppe:
- Mineralöle, Mineralwachse
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Eine Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid, Dicaprylylether, Dicaprylyl Carbonat, Butylen Gylcol Dicaprylat/Dicaprat.

Besonders vorteilhaft sind Mischungen aus C₁₂₋₁₅-Alkylbenzoat und 2-Ethylhexylisostearat, Mischungen aus C₁₂₋₁₅-Alkylbenzoat und Isotridecylisononanoat sowie Mischungen aus C₁₂₋₁₅-Alkylbenzoat, 2-Ethylhexylisostearat und Isotridecylisononanoat.

Von den Kohlenwasserstoffen sind Paraffinöl, Squalan und Isoparaffin vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) oder Dimethicon als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wäßrige Phase der erfindungsgemäßen Zubereitungen enthält gegebenenfalls vorteilhaft
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Es ist erfindungsgemäß vorteilhaft, außer den erfindungsgemäßen Kombinationen weitere öllösliche UVA-Filter und/oder UVB-Filter in der Lipidphase und/oder weitere wasserlösliche UVA-Filter und/oder UVB-Filter in der wäßrigen Phase einzusetzen.

Vorteilhaft können die Lichtschutzformulierungen erfindungsgemäß weitere Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 20 Gew.-%, insbesondere 1 bis 15 Gew.-% beträgt, bezogen auf das Gesamtgewicht der Zubereitungen, um kosmetische Zubereitungen zur Verfügung zu stellen, welche die Haut vor dem gesamten Bereich der ultravioletten Strahlung schützen. Sie können auch als Sonnenschutzmittel dienen.

Die weiteren UVB-Filter können öllöslich oder wasserlöslich sein. Vorteilhafte öllösliche UVB-Filtersubstanzen sind z.B.:
- 4-Aminobenzoësäure-Derivate, vorzugsweise 4-(Dimethylamino)-benzoësäure(2-ethylhexyl)ester, 4-(Dimethylamino)benzoësäureamylester;
- Ester der Benzalmalonsäure, vorzugsweise 4-Methoxybenzalmalonsäuredi(2-ethylhexyl)ester;
- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Methylidencampherderivate, vorzugsweise 4-Methylbenzylidencampher, Benzylidencampher;
- Triazinderivate, vorzugsweise 4,4',4"-(1,3,5-Triazin-2,4,6-triylimino)-tris-benzoesäuretris-(2-ethylhexylester) [UVASorb HEB (Sigma 3V)]
Vorteilhafte wasserlösliche UVB-Filtersubstanzen sind z.B.:
- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate des 3-Benzylidencamphers, wie z.B. 4-(2-Oxo-3-bornylidenmethyl)benzolsulfonsäure,2-Methyl-5-(2-oxo-3-bornylidenmethyl)sulfonsäure und deren Salze.

Die Liste der genannten UVB-Filter, die zusätzlich im Sinne der vorliegenden Erfindung eingesetzt werden können, soll selbstverständlich nicht limitierend sein.

Es kann auch von Vorteil sein, UVA-Filter einzusetzen, die bisher üblicherweise in kosmetischen Zubereitungen enthalten sind. Es können die für die UVB-Kombination verwendeten Mengen eingesetzt werden. Vorteilhafte UVA-Filter sind z.B.:
- 1,4-Phenylendimethincamphersulfonsäurederivate wie z.B. 3,3'-(1,4-Phenylendimethin)-bis-(7,7-dimethyl-2-oxobicyclo[2.2.1]-heptan-1-methamsulfonsäure und ihre Salze
- 1,3,5-Triazinderivate wie 2,4-Bis{[(2-ethylhexyloxy)-2-hydroxy)-phenyl}-6-(4-methoxyphenyl)-1,3,5)-triazin [Tinosorb S von Ciba]
- Dibenzoylmethanderivate, vorzugsweise 4-lsopropyldibenzoylmethan, 4-(tert.-Butyl)-4'-methoxydibenzoylmethan

Ferner kann erfindungsgemäß gegebenenfalls von Vorteil sein, die Zubereitungen mit weiteren UVA- und/oder UVB-Filtern zu versehen, beispielsweise bestimmten Salicylsäurederivaten wie

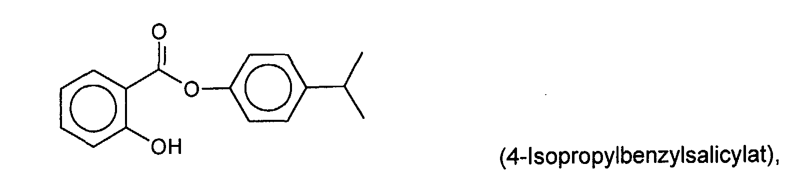

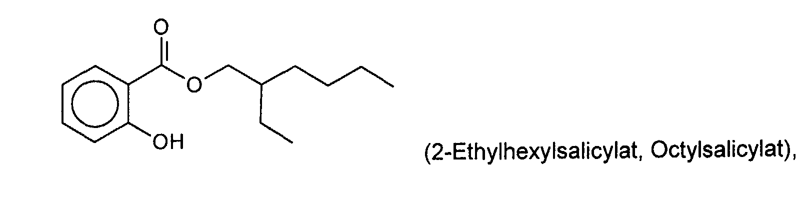

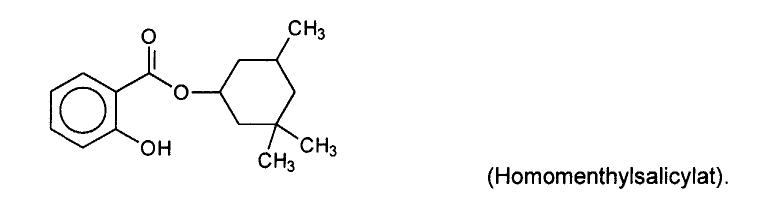

Die Gesamtmenge an einem oder mehreren Salicylsäurederivaten in den fertigen kosmetischen oder dermatologischen Zubereitungen wird vorteilhaft aus dem Bereich von 0,1 - 15,0 Gew.-%, bevorzugt 0,5 - 10,0 Gew.-% gewählt, bezogen auf das Gesamtgewicht der Zubereitungen. Wenn Ethylhexylsalicylat gewählt wird, ist es von Vorteil, dessen Gesamtmenge aus dem Bereich von 0,1 - 10,0 Gew.-%, bevorzugt 0,5 - 8 Gew.-% zu wählen. Wenn Homomenthylsalicylat gewählt wird, ist es von Vorteil, dessen Gesamtmenge aus dem Bereich von 0,1 - 10,0 Gew.-%, bevorzugt 0,5 - 5,0 Gew.-% zu wählen.

Noch eine weiterere erfindungsgemäß vorteilhaft zu verwendende zusätzliche Lichtschutzfiltersubstanz ist das Ethylhexyl-2-cyano-3,3-diphenylacrylat (Octocrylen), welches von BASF unter der Bezeichnung UVINUL® N 539 erhältlich ist und sich durch folgende Struktur auszeichnet:

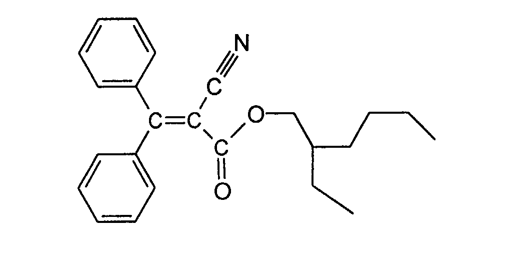

Das erfindungsgemäße Verhältnis von UV-Filter zu Selbstbräuner beträgt 0,1 : 1 bis 1 : 0,1.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Beispiele:

| **1. O/W Emulsionen** | | | | | | | |
|---|---|---|---|---|---|---|---|
| | **1** | **2** | **3** | **4** | **5** | **6** | **7** |
| Dihydroxyaceton | 4,0 | 6,0 | 5,0 | 4,0 | 3,0 | 5,0 | 2,0 |
| Glycerinmonostearat SE | | | 1,5 | | | 0,5 | 4,0 |
| Glyceryl Stearat Citrat | 2,0 | | | | | 3,0 | |
| PEG-40 Rizinus Öl | | 2,0 | | | | | |
| Glyceryl Stearat | | | | 5,0 | | | |
| PEG-30 Stearat | | | | 1,5 | | | |
| Natrium Stearat | | 0,5 | | | | | |
| Stearinsäure | | | | | 2,0 | | 2,0 |
| PEG-40 Stearat | | | | | | 0,5 | |
| Cetyl Phosphat | 1,0 | | | | 2,5 | | |
| Cetearyl Alkohol | | 1,0 | 1,0 | | | | |
| Cetyl Alkohol | | | 2,0 | | 2,0 | 2,5 | |
| Polyglyceryl-3 Methylglucose Distearat | | | | | 3,5 | | |
| Glyceryl Lanolat | 0,5 | | | | 0,5 | | |
| Lanolin Alkohol | | | | | 0,5 | | |
| Myristyl Alkohol | | | | | 1,0 | | |
| Natrium Cetearyl Sulfat | | 1,0 | 1,0 | | | | |
| Butyl Methoxydibenzoylmethan | | | 2,0 | | 1,0 | 2,0 | |
| Bis-Ethylhexyloxyphenol Methoxyphenyl Triazin | | 1,5 | | | 2,0 | | |
| Dinatrium Phenyl Dibenzimidazol Tetrasulfonat | 1,0 | 2,0 | | 1,5 | | | 0,5 |
| Methylen Bis-Benztriazolyl Tetramethylbutylphenol | 3,0 | | 4,0 | 1,0 | 2,0 | 3,0 | |
| Drometrizol Trisiloxan | | | | 1,0 | 2,0 | 0,5 | 1,0 |
| Octocrylen | | 5,0 | | | 3,0 | | 2,5 |
| Ethylhexyl Methoxycinnamat | | | 2,0 | 4,0 | | | |
| Ethylhexyl Salicylat | 4,0 | | 1,5 | | | | |
| Diethylhexyl Butamidotriazon | 2,0 | 1,5 | 2,0 | | | 3,0 | |
| Ethylhexyl Triazon | | | | 2,0 | 1,0 | | 1,0 |
| Terephtaliden Dicampher Sulfonsäure | | 0,1 | | | | 1 | |
| Titandioxid | | | | 0,50 | | | 0,50 |
| Octyldodecanol | 5,00 | | 3,00 | | 3,50 | | 3,00 |
| C12-15 Alkyl Benzoat | | | 5,00 | | | | |
| Dicaprylyl Ether | | 5,00 | | | | 5,00 | |
| Capryl/Caprin Triglycerid | | | 2,00 | | 4,00 | | |
| Dicaprylyl Carbonat | | | | | | | |
| Dimethicon | | | | 3,00 | | 2,00 | |
| Cyclomethicon | | | 3,00 | | | | |
| Shea Butter | | 0,50 | | | 1,00 | | |
| Trinatrium EDTA | 1,00 | | | | | 1,00 | 0,50 |
| Glycerin | | | 3,00 | | 3,00 | | 3,00 |
| Xanthan Gummi | | | 0,50 | | 1,00 | | |
| Natrium Carbomer | | 0,50 | | | | 0,50 | |
| Vitamin E Acetat | 0,50 | | 0,50 | | 0,50 | | |
| Jodopropynyl Butylcarbamat | 0,10 | | | 0,10 | | 0,10 | |
| Methylparaben | 0,30 | | 0,25 | 0,03 | 0,25 | 0,25 | 0,30 |
| Phenoxyethanol | | 0,50 | 0,25 | | 0,25 | 0,25 | |
| Ethanol | | | 5,00 | | 3,00 | | 3,50 |
| Farbstoffe (öl- und/oder wasserlöslich) | 0,01 | | | 0,03 | 0,05 | | |
| Trinatrium EDTA | 0,4 | 0,25 | | 0,3 | | 0,4 | 0,25 |
| Citrat-Puffer | q.s. | q.s. | q.s. | q.s. | q.s. | q.s. | q.s. |
| Parfüm | 0,3 | 0,2 | 0,45 | 0,3 | 0,2 | | 0,3 |
| Wasser | ad. 100 | ad. 100 | ad. 100 | ad. 100 | ad. 100 | ad. 100 | ad. 100 |

| **2. Hydrodispersionen** | | | | | |
|---|---|---|---|---|---|
| | **1** | **2** | **3** | **4** | **5** |
| Dihydroxyaceton | 3,0 | 7,0 | 5,0 | 4,0 | 5,0 |
| Ceteareth-20 | | 0,5 | | | 1,5 |
| Cetyl Alkohol | | | 0,75 | | |
| Natrium Carbomer | 0,4 | 0,2 | | | |
| Acrylat/C10-30 Alkyl Acrylat Crosspolymer | 0,15 | | 0,4 | | 0,2 |
| Xanthan Gummi | | 0,5 | | 0,8 | 0,4 |
| Butyl Methoxydibenzoylmethan | 1,0 | | 0,5 | | |
| Bis-Ethylhexyloxyphenol Methoxyphenyl Triazin | | 2,0 | 1,0 | | |
| Dinatrium Phenyl Dibenzimidazol Tetrasulfonat | 0,5 | 1,0 | | 2,0 | |
| Methylen Bis-Benztriazolyl Tetramethylbutylphenol | | 2,0 | | 1,0 | 4,0 |
| Drometrizol Trisiloxan | 1,5 | | 1,0 | 0,2 | |
| Octocrylen | | 4,0 | 2,0 | | |
| Ethylhexyl Methoxycinnamat | 2,0 | | | 7,0 | |
| Ethylhexyl Salicylat | | 4,5 | 4,0 | | |
| Diethylhexyl Butamidotriazon | 3,0 | | | | 1,5 |
| Ethylhexyl Triazon | 2,0 | | 1,5 | | 3,0 |
| Terephtaliden Dicampher Sulfonsäure | | 1,0 | 0,5 | | |
| Titandioxid | | 1,0 | | 2,0 | 0,5 |
| Zinkoxid | | 0,5 | 0,5 | | |
| C12-15 Alkyl Benzoat | 5,0 | | | | 9,0 |
| Dicaprylyl Ether | | 1,0 | 2,0 | | |
| Butylenglycol Dicaprylat/Dicaprat | 3,0 | | | | 1,0 |
| Dicaprylyl Carbonat | 2,0 | 4,0 | | | |
| Dimethicon | | | 1,0 | 2,0 | |
| Phenyltrimethicon | | | 0,5 | | 2,0 |
| Shea Butter | 2,0 | | | | |
| PVP Hexadecen Copolymer | | 0,5 | | 1,0 | |
| Octoxyglycerin | 0,3 | 0,3 | | 0,5 | |
| Glycerin | 5 | 7,5 | 10 | | 7,5 |
| Glycin Soja | | | | 1,5 | |
| Vitamin E Acetat | 0,5 | 1,5 | 0,3 | | 1,0 |
| Polyurethan | | 1,0 | | | |
| DMDM Hydantoin | | 0,1 | | | |
| Konkaben LMB ® | | | | | 0,2 |
| Methylparaben | | | 0,3 | | 0,3 |
| Phenoxyethanol | | 1,0 | 0,4 | | |
| Ethanol | 4,0 | | 2,0 | 5,0 | |
| Parfüm | 0,4 | | 0,2 | | 0,2 |
| Trinatrium EDTA | | 0,2 | 0,3 | | 0,4 |
| Farbstoff | 0,05 | | | 0,1 | |
| Citrat-Puffer | q.s. | q.s. | q.s. | q.s. | q.s. |
| Wasser | ad. 100 | ad. 100 | ad. 100 | ad. 100 | ad. 100 |

| **3. W/O Emulsionen** | | | | | |
|---|---|---|---|---|---|
| | **1** | **2** | **3** | **4** | **5** |
| Dihydroxyaceton | 3,0 | 6,0 | 5,0 | 4,0 | 5,0 |
| Cetyldimethicon Copolyol | 2,5 | 0,75 | | 3,5 | |
| Polyglyceryl-2-dipolyhydroxystearat | 1,0 | | 3,0 | | 3 |
| PEG-30-dipolyhydroxystearat | | 2,0 | | | 2,5 |
| Butyl Methoxydibenzoylmethan | 0,5 | | 2,0 | 1,0 | |
| Bis-Ethylhexyloxyphenol | 0,5 | 1,0 | | 0,5 | |
| Methoxyphenyl Triazin | | | | | |
| Dinatrium Phenyl Dibenzimidazol Tetrasulfonat | 1,5 | 1,0 | | | 2,0 |
| Methylen Bis-Benztriazolyl Tetramethylbutylphenol | | | 4,0 | | 4,0 |
| Drometrizol Trisiloxan | | 1,0 | | 2,5 | |
| Octocrylen | | 4,0 | | 7,0 | 5,0 |
| Ethylhexyl Methoxycinnamat | 7,0 | 4,0 | | | |
| Ethylhexyl Salicylat | | | 2,0 | | |
| Diethylhexyl Butamidotriazon | 2,0 | 3,0 | | 2,5 | |
| Ethylhexyl Triazon | 2,0 | | 3,5 | | |
| Terephtaliden Dicampher Sulfonsäure | | | | 0,4 | 1,0 |
| Titandioxid | | 2,0 | 3,0 | | |
| Zinkoxid | 0,5 | | | | 1,5 |
| Mineralöl | 6,0 | 5,5 | 8,5 | | |
| C12-15 Alkyl Benzoate | 2,0 | | 3,0 | | |
| Dicaprylyl Ether | | 1,5 | | | 4,0 |
| Butylenglycol Dicaprylat/Dicaprat | 7,5 | 6,0 | | | 5,0 |
| Dicaprylyl Carbonat | | | | 2,0 | |
| Dimethicon | | 2,0 | | 6,0 | |
| Cyclomethicon | 1,5 | | | 5,0 | |
| Shea Butter | 0,75 | | 2,0 | | |
| PVP Hexadecen Copolymer | | 0,5 | | 0,75 | |
| Octoxyglycerin | 0,3 | | | | 0,75 |
| Glycerin | 10 | | 7,5 | 5,0 | |
| Butylen Glycol | | 7,5 | | 4,0 | 10 |
| Glycin Soja | 1,0 | 1,5 | | | 0,5 |
| Magnesium Sulfat | 0,7 | | 0,8 | 1,2 | |
| Magnesium Chlorid | | 0,5 | | | 0,8 |
| Vitamin E Acetat | 0,5 | 1,0 | 0,75 | 0,5 | |
| DMDM Hydantoin | | | 0,1 | | |
| Methylparaben | | 0,4 | 0,3 | | 0,3 |
| Phenoxyethanol | | 1,0 | | 0,5 | |
| Ethanol | 2,0 | | 4,0 | 2,5 | |
| Trinatrium EDTA | | 0,7 | 0,3 | | 0,5 |
| Parfüm | 030 | | 0,40 | 0,20 | |
| Citrat-Puffer | q.s. | q.s. | q.s. | q.s. | q.s. |
| Wasser | ad. 100 | ad. 100 | ad. 100 | ad. 100 | ad. 100 |

| **4. PIT - Sprays** | | | | | |
|---|---|---|---|---|---|
| | **1** | **2** | **3** | **4** | **5** |
| Dihydroxyaceton | 3,0 | 6,0 | 5,0 | 4,0 | 5,0 |
| Glycerinmonostearat SE | 4,0 | | | 3,5 | |
| Ceteareth-12 | | 0,5 | | 0,2 | |
| Ceteareth-20 | 3,0 | | | 2,5 | |
| Ceteareth-30 | | 2,5 | | | |
| Isoceteth-20 | | | 5,4 | | 4,0 |
| Glyceryl Isostearat | | 4,5 | 3,5 | | 5,0 |
| Stearyl Alkohol | | 2,0 | | 0,75 | |
| Cetyl Alkohol | 2,0 | | 2,5 | | 1,5 |
| Butyl Methoxydibenzoylmethan | | 1,5 | | 0,3 | |
| Bis-Ethylhexyloxyphenol Methoxyphenyl Triazin | 0,75 | | | 1,0 | 1,7 |
| Dinatrium Phenyl Dibenzimidazole Tetrasulfonat | 2,0 | | 2,5 | 0,5 | |
| Methylen Bis-Benztriazolyl Tetramethylbutylphenol | | 4,0 | | 2,5 | |
| Drometrizol Trisiloxan | 0,7 | 1,0 | | 2,0 | 1,5 |
| Octocrylen | 2,0 | | 5,0 | | 4,0 |
| Ethylhexyl Methoxycinnamat | | 4,0 | 2,5 | 7,5 | |
| Ethylhexyl Salicylat | | 2,5 | 3,0 | | |
| Diethylhexyl Butamidotriazon | 1,0 | | | 1,5 | |
| Ethylhexyl Triazon | 2,0 | | 1,0 | | |
| Terephtaliden Dicampher Sulfonsäure | | 0,2 | | 1,0 | |
| Phenylbenzmidazol Sulfonsäure | 1,0 | 2,0 | | | 2,0 |
| C12-15 Alkyl Benzoat | 2,0 | | 2,0 | 2,0 | |
| Dicaprylyl Ether | | 1,0 | | | |
| Butylenglycol Dicaprylat/Dicaprat | | | 2,0 | 3,0 | |
| Dicaprylyl Carbonat | 4,0 | | | 0,5 | 5,0 |
| Dimethicon | | 0,75 | | | |
| Phenyltrimethicon | 1,5 | | 1,0 | 0,75 | |
| Shea Butter | | 2,0 | 2,0 | | |
| PVP Hexadecen Copolymer | 0,5 | | 1,0 | | 1,0 |
| Tricontanyl PVP | | | | 0,8 | 0,5 |
| Glycerin | 10 | | 7,5 | 5,0 | 5,0 |
| Butylen Gylcol | | 10 | 3,5 | | |
| Vitamin E Acetat | 0,5 | 1,0 | | 0,5 | |
| DMDM Hydantoin | | | | | 0,1 |
| Konkaben LMB ® | | | 0,18 | 0,2 | |
| Methylparaben | 0,3 | | 0,2 | | |
| Phenoxyethanol | | 1,0 | 0,5 | 1,0 | 0,0 |
| Ethanol | 3,0 | 2,0 | | | |
| Parfüm | 0,2 | 0,2 | 0,5 | 0,3 | 0,4 |
| Trinatrium EDTA | 0,25 | 0,5 | | 0,3 | 0,1 |
| Farbstoff | 0,01 | | 0,03 | 0,15 | |
| Citrat - Puffer | q.s. | q.s. | q.s. | q.s. | q.s. |
| Wasser | ad. 100 | ad. 100 | ad. 100 | ad. 100 | ad. 100 |

| **5. Feststoffstabilisierte Emulsionen** | | | | | |
|---|---|---|---|---|---|
| | **1** | **2** | **3** | **4** | **5** |
| Dihydroxyaceton | 5,0 | 4,0 | 5,0 | 7,0 | 5,0 |
| Mineralöl | 15 | | 7,5 | 9,0 | |
| Octyldodecanol | | 9,0 | 6,0 | | 10,0 |
| Capryl/Caprin Triglycerid | 5,5 | 5,0 | | | |
| C12-15- Alkyl Benzoat | | | 4,0 | 7,0 | |
| Butylen Glycol Dicaprylat/Dicaprat | 7,0 | 9,0 | | 5,0 | 9,0 |
| Dicaprylyl Ether | | 3,0 | 2,5 | | |
| Dicaprylyl Carbonat | 4,5 | | 5,0 | | 3,0 |
| Hydroxyoctacosanyl Hydroxystearat | 2,0 | | 1,0 | 1,5 | 2,0 |
| Disteardimonium Hectorit | 1,5 | 1,75 | 3,5 | 2,0 | 0,7 |
| Vaseline | | 2,0 | | 0,5 | |
| Hydroxypropyl Methylcellulose | 0,2 | | 0,15 | | |
| Dimethicon | | 1,5 | | | 4,0 |
| Butyl Methoxydibenzoylmethan | 0,5 | | 1,0 | 0,7 | |
| Bis-Ethylhexyloxyphenol Methoxyphenyl Triazin | 0,5 | | 1,0 | | 2,0 |
| Dinatrium Phenyl Dibenzimidazol Tetrasulfonat | 2,0 | 2,5 | 1,5 | | 1,5 |
| Methylen Bis-Benztriazolyl Tetramethylbutylphenol | | 4,0 | 1 | 3,0 | |
| Drometrizol Trisiloxan | | | 1,5 | 2,0 | 0,5 |
| Octocrylen | 5,0 | | 2,5 | 7.0 | |
| Ethylhexyl Methoxycinnamat | | 5,0 | | | 5,0 |
| Ethylhexyl Salicylat | 0,5 | | | | |
| Diethylhexyl Butamidotriazon | | 1,0 | 2,5 | | 0,5 |
| Ethylhexyl Triazon | | 3,0 | | 1,0 | 2,5 |
| Terephtaliden Dicampher Sulfonsäure | 0,2 | | 1,0 | | |
| Phenylbenzmidazol Sulfonsäure | | 1,5 | | | |
| Titandioxid + Aluminumoxid + Simethicon + Wasser | 4,0 | 3,5 | | 2,5 | |
| Titandioxid + Trimethoxycaprylylsilan | | | 3,0 | 2,0 | 3,0 |
| Zinkoxid | 4,0 | | 2,5 | | 3,0 |
| Silica Dimethyl Silylat | | 1,0 | | 0,7 | |
| Bornitrid | | 2,0 | 3,5 | | |
| Stärke/-Natriummetaphosphat-Polymer | 1,5 | | | | 3,0 |
| Tapioca Stärke | | 1,0 | 0,7 | | |
| Natrium Chlorid | 1,0 | 1,5 | | 0,7 | |
| Magnesium Sulfat | | | 1,0 | | 0,5 |
| Glycerin | 5,0 | 10,0 | 7,5 | 3,5 | 5,0 |
| Trinatrium EDTA | | 0,4 | 0,25 | 0,3 | 0,7 |
| Methylparaben | 0,3 | | | 0,3 | 0,3 |
| Propylparaben | 0,1 | | 0,3 | 0,2 | |
| Phenoxyethanol | 0,5 | | | | 1,0 |
| Hexamidin Diisethionat | | 0,01 | | | |
| Diazolidinyl Harnstoff | | | 0,02 | | |
| Ethanol | 2,0 | | | 5,0 | |
| Farbstoff | | 0,1 | | 0,02 | 0,04 |
| Parfüm | q.s. | q.s. | q.s. | q.s. | q.s. |
| Wasser | Ad 100 | Ad 100 | Ad 100 | Ad 100 | Ad 100 |

## Patentansprüche

1. Kosmetische Formulierungen, die mindestens einen UV-Filter mit Benzoazolyl-Gruppen und/oder mindestens einen UV-Filter mit Benzodiazolyl-Gruppen und/oder mindestens einen UV-Filter auf Basis eines Benzotriazolderivates und mindestens eine Selbstbräunungssubstanz enthalten.

2. Kosmetische Formulierungen nach Anspruch 1, welche als Selbstbräunungssubstanz 1,3-Dihydroxyaceton (DHA) enthalten.

3. Kosmetische Formulierungen nach einem der Ansprüche 1 oder 2, welche als Selbstbräunungssubstanz 1,3-Dihydroxyaceton (DHA) in einer Konzentration von 0,1 bis 10 Gewichts-%, bezogen auf das Gesamtgewicht der Formulierung enthalten.

4. Kosmetische Formulierungen nach einem der Ansprüche 1 bis 3, die weitere organisch-chemische UV-Filter enthalten.

5. Kosmetische Formulierungen nach einem der Ansprüche 1 bis 4, die zusätzlich anorganische Pigmente als UV-Sonnenschutzfilter enthalten.

6. Kosmetische Formulierungen nach einem der Ansprüche 1 bis 5, die als UV-Filter Benzotriazole in pigmentärer Form enthalten.

7. Kosmetische Formulierungen nach einem der vorherigen Ansprüche, die als UV-Filter Benzotriazole in gelöster Form enthalten.

8. Kosmetische Formulierungen nach einem der vorherigen Ansprüche, welche UV-Filter mit Benzotriazol-Gruppen in einer Konzentration von 0,1 bis 10 Gewichts-% bevorzugt in einer Konzentration von 0,2 bis 5 Gewichts-%, jeweils bezogen auf das Gesamtgewicht der Formulierung, enthalten.

9. Kosmetische Formulierungen nach einem der vorherigen Ansprüche, welche UV-Filter mit Benzoazolyl-Gruppen in einer Konzentration von 0,1 bis 10 Gewichts-%, bevorzugt in einer Konzentration von 0,2 bis 5 Gewichts-%, jeweils bezogen auf das Gesamtgewicht der Formulierung, enthalten.

10. Kosmetische Formulierungen nach einem der vorherigen Ansprüche, welche UV-Filter mit Benzodiazolyl-Gruppen in einer Konzentration von 0,1 bis 10 Gewichts-%, bevorzugt in einer Konzentration von 0,2 bis 5 Gewichts-%, jeweils bezogen auf das Gesamtgewicht der Formulierung, enthalten.

11. Kosmetische Formulierungen, deren Verhältnis von UV-Filter zu Selbstbräuner 0,1 : 1 bis 1 : 0,1 beträgt.

12. Formulierungen nach einem der vorherigen Ansprüche, dahingehend dass sie öllösliche und/oder wasserlösliche Farbstoffe enthalten.

13. Formulierungen nach einem der vorherigen Ansprüche, dahingehend dass sie pigmentäre Farbstoffe enthalten.

14. Verwendung von Formulierungen nach einem der vorherigen Ansprüche zur Hautbefeuchtung.

15. Verwendung von Formulierungen nach einem der Ansprüche 1 bis 13 zum Schutz vor Hautalterung.

16. Verwendung von Formulierungen nach einem der Ansprüche 1 bis 15 als Sonnenschutzmittel und/oder Selbstbräunungsmittel.
